# EUROPEAN PATENT SPECIFICATION

(11) **EP 3 758 632 B1**
(45) Date of publication and mention of the grant of the patent: **04.08.2021**
(21) Application number: 19719372.5
(22) Date of filing: 26.02.2019
(51) Int. Cl.: A61B 17/68

(54) **DEVICE FOR FIXING A CRANIOTOMY OPERCULUM TO THE CRANIAL VAULT TO BE INSERTED IN THE CRANIOTOMY CUT**
VORRICHTUNG ZUM BEFESTIGEN EINES KRANIOTOMIEOPERCULUMS AN DER SCHÄDELKALOTTE ZUM EINSETZEN IN EINEN KRANIOTOMIESCHNITT
DISPOSITIF DE FIXATION D'UN OPERCULE DE CRANIOTOMIE À LA VOÛTE CRÂNIENNE DESTINÉ À ÊTRE INTRODUIT DANS LA DÉCOUPE DE CRANIOTOMIE

(30) Priority: 02.03.2018 IT 201800003225
(43) Date of publication of application: 06.01.2021
(73) Proprietor: Ntplast S.r.l., 00198 Roma (IT)
(72) Inventor: GAZZANI, Igino Romolo, 00198 Rom (IT); FALCOLINI, Luca, 00198 Rom (IT)
(74) Representative: Garavelli, Paolo
(86) International application number: PCT/IT2019/000016
(87) International publication number: WO 2019/167092

(56) References cited:
- WO-A1-2013/136348
- WO-A1-2014/174538
- US-A1- 2013 282 011

## Description

The present invention refers to a device for stably fixing a craniotomy operculum to the cranial vault, to be placed along a craniotomy cut.

A craniotomy is a surgical intervention for opening the skull which is made through an incision and cutting of the cranial vault. The craniotomy is a compulsory procedure for any intra-cranial intervention.

The procedure for opening the cranial vault, for performing intradural operations, provides for cutting the skin and the subcutaneous part with a scalpel. Once having exposed the bone, the periostium is cut with an electric scalpel and detached from the bone with a suitable instrument. Once si bone is adequately exposed, one or more holes are drilled with a special drill. After having detached the menino below the drilled holes, an instrument, called craniotomie, is used, with which a free bone limb is created, which completely exposes the dura mater (meninx) below.

At that time, the craniotomy ends. At the end of the intradural procedure, the craniotomy operculum is put again in place and ensured to the free edges through suitable fastening devices. The periostium is sutured again, as well as the other planes above. Obviously, between the craniotomy operculum and tthe bone of the cranial vault, a slit remains having the width of the blade used for cutting, the slit being afterwards closed by the bone tissue which is spontaneously rebuilt.

The use of fastening devices of the type disclosed in document EP-B1-2988687, of the same Applicant, allows, in general, an aesthetically valid closure, avoiding that the craniotomy operculum can be projected, encased, inclined or rotated.

The device disclosed in document EP-B1-2988687B1 is essentially composed of a tensioner which is inserted in the slid existing between the bone of the cranial vault and the craniotomy operculum, and first and second closing means designed to fasten the craniotomy operculum in its correct position, in particular without projections or recesses. The first closing means comprise:
- a pair of elongated elastic arms, operatively connected to the lower part of the tensioner, namely the part oriented towards inside the skull, each of the elongated elastic arms ending in two small transverse arms;
- a lower base of the tensioner, in turn equipped with the small transverse arms which are inserted in the epidural space (X) between cranial vault and dura mater.

The second closing means comprise a plate sliding assembled on the tensioner, the plate being ablle to be pressed against outside the cranial vault so that a fixing is obtained through the tightening against the cranial bones from inside and from outside, the tightening being enabled by the elasticity of the fastening device.

When the fastening device is in its operating position, the elongated elastic arms are pressed against the internal distal cranial surface and the plate is pressed against the external surface of the cranial vault.

The prior art device, though allowing an optimum fastening of the craniotomy operculum, has however an inconvenience, since the lower base, which must be inserted in the epidural space between dura mater and cranial vault bone, has such an encumbrance as to require the detachment of excessive parts of the epidural space between dura mater and cranial vault, this operation being very delicate and implying high risks of bleeding. On the other hand, the assembling of the device without the use of the lower base, though being possibile, has also inconveniences due to the fact that the shape of the elongated elastic arms has an edgy bit, with the risk of tearing, when inserting it in the epidural space, the dura mater, with high risks of bleeding.

Document US2013/282011 A1 discloses a fastening device according to the preamble of claim 1.

Object of the present invention is solving the above prior art problems, by providing a device for fixing a craniotomy operculum to a cranial vault, designed to be positioned in a craniotomy crack, comprising:
- at least one supporting tensioner;
- first closing means operatively connected to the supporting tensioner;
- second closing means designed to be fastened to the supporting tensioner for completing its closure;
- at least one handle removably connected to the supporting tensioner and designed to handle the device of closure to take it from its rest position to its operating fastening position, wherein the first closing means are connected to a distal end of the supporting tensioner and the second closing means are designed to be fastened to the supporting tensioner in a position near the distal end of the supporting tensioner, so that, when the fastening device is in its operating position, the first closing means are placed against the internal distal cranial surface and the second closing means are placed against the external cranial surface, the first closing means comprising at least two elongated elastic arms operatively connected to the lower end of the tensioner, each of the elongated elastic arms ending in two small elongated arms transversally placed with respect to the elongated elastic arms; the small transverse arms are shaped as a tapered slider, suitable to enable a wedging of the same small transverse arm and its following sliding in the epidural space above the dura mater and the cranial vault, generating only a minimum disconnection of the dura mater and reducing to a minimum the danger of bleeding.

The new small transverse arms guarantee the application of the device along the craniotomy cut with an insertion without traumas, evne next to the line of the brain sinuses: this greatly widens its field of application. The new geometry evoids detaching excessive parts of dura mater, strongly reducing the risk of bleeding.

Preferred embodiments and non-trivial variations of the present invention are the subject matter of the dependent claims.

It is intended that all enclosed claims are an integral part of the present description.

It will be immediately obvious that numerous variations and modifications (for example related to shape, sizes, arrangements and parts with equivalent functionality) can be made to what is described, without deparing from the scope of the invention as appears from the enclosed claims.

The present invention will be better described by some preferred embodiments thereof, provided as a non-limiting example, with reference to the enclosed drawings, in which:
Figure 1 shows an embodiment of the device for stably fixing a craniotomy operculum to the cranial vault, according to the present invention;
Figure 2 shows an example of use of the device for stably fixing a craniotomy operculum to the cranial vault, according to the present invention; and
Figure 3 shows details of the device according to the present invention.

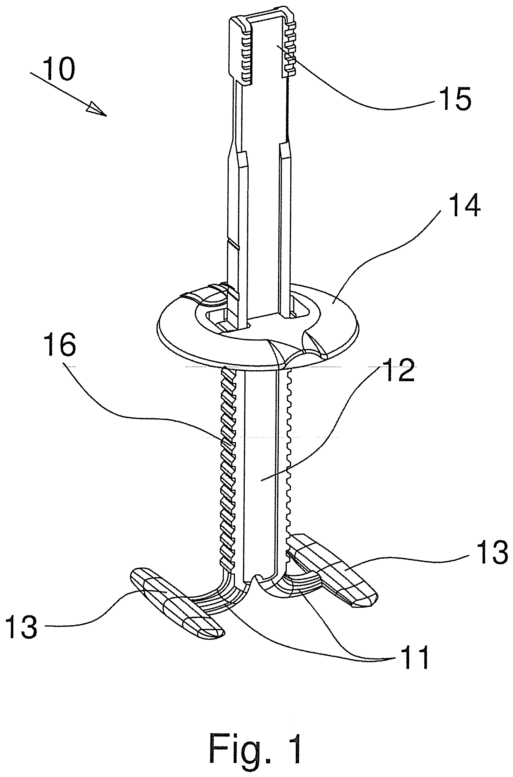

With reference to Figure 1, the device for fixing a craniotomy operculum to the cranial vault, to be inserted in the craniotomy cut, according to the present invention, is globally designated with reference number (10). This device (10) comprises a pair of elongated elastic arms (11), connected to the lower part of a tensioner (12) and ending into tiny elongated arms (13) arranged transversally with respect to the axis of the elongated elastic arms (11), and an upper plate (14), designed to be inserted in the upper part of the tensioner (12). The tensioner (12) is equipped with a handle (15) in its upper part and with a plurality of fastening elements (16) (which can be, for example, small teeth as shown in the enclosed Figures). The fastening elements (16) are used for blocking the upper plate (14) in its operating position.

Due to the resiliency of the arms (11), the tensioner (12), which connects the elastic arms (11) and the upper plate (14), allows a fine adjustment of the position of the plate (14) depending on the thickness of the cranial vault bone.

The arrangement of the device (10) therefore provides that the first closing means (11, 13) are connected to the lower distal end of the tensioner (12) and that the closure plate (14) is designed to be fastenet to the lift of the tensioner (12), in a position near the distal end of the tensioner (12), so that, when the device (10) is in its operating fastening position, the first closing means (11, 13) are pressed against the internal distal cranial surface while the second closing means (14) are pressed against the external surface of the cranial vault.

Accordino to a preferred embodiment, the device (10) is made of an injection-molded biocompatible elastic plastic material, for example PEEK (Polyether Ether Ketone).

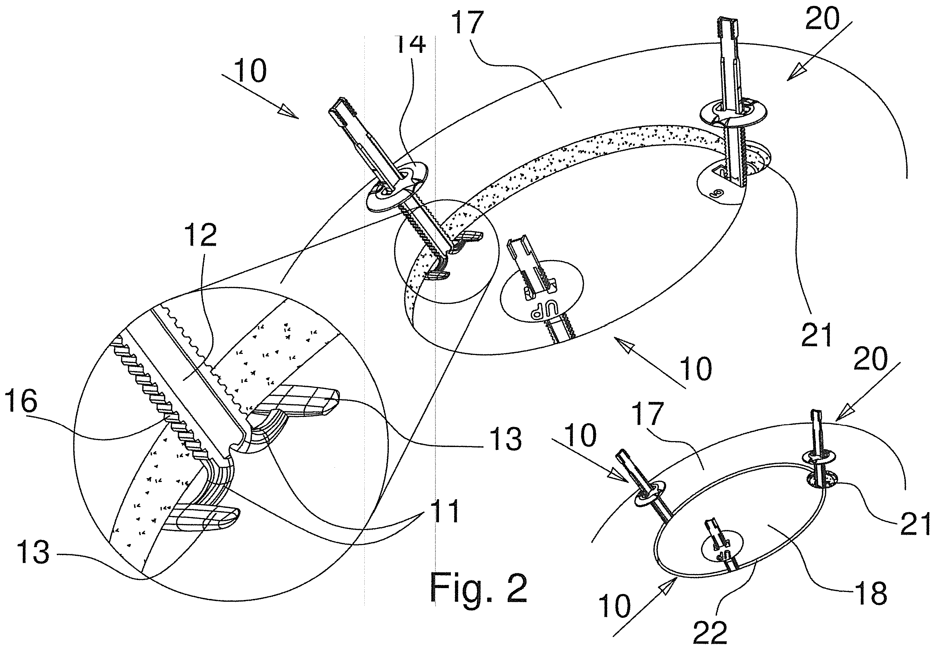

Figure 2 shows an example of use of the fastening device (10) according to the invention.

Examining more in detail the use modes of the fastening device (10), the operator prepares the right amount of devices (10) suitable for fixing a craniotomy operculum (18), choosing the more suitable number of devices (10), based on the sizes and the shape of the craniotomy. In the case as shown, there are two fastening devices (10) according to the invention, to be applied to the craniotomy crack (22), and a fastening device (20), according to EP-B1-2988687, for its application next to the hole (21), obtained for performing the craniotomy.

The bone operculum (18) is re-positioned as shown in Figure 2. In this way, the tensioners (12) of the fastening devices (10, 20) are projected towards outside, respectively from the craniotomy hole (21) and from the craniotomy crack (22).

Afterwards, the surgeon, after having checked the correct position of the operculum (18), slighlt tractions the tensioner (12), grasping it by its handle (15), and then inserts the plates (14) and tightens them against the external part of the cranial bone, thereby performing the blockage of the operculum (18). In this configuration, the elastic arms (11) are slightly flexed to be able to exert the correct thrust against the internal part of the cranial vault.

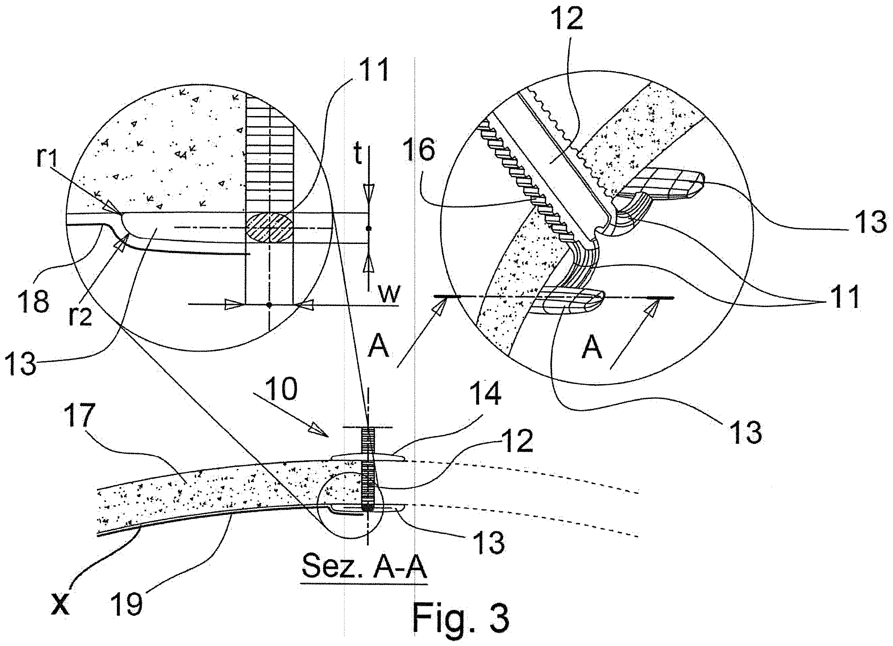

When positioning the fastening devices (10) of the invention, the surgeon must be careful of checking the correct isnertion of the small transverse arms (13) into the epidural space (X) between dura mater (19) and cranial vault (17) bone, as shown in particular in Figure 3. During this operation, it is necessary to keep the elastic arms (11) aligned along the direction of the craniotomy crack (22) and the tensioner (12) of the fastening device (10) perpendicular to the cranial surface. In this way, the following positioning of the craniotomy operculum (18) occurs by resting the lower surface of the operculum (18) on the small transverse arms (13) and the following tightening of the plate (14) against the external surface of the cranial vault (17) will ensure the perfect alignment of the bone edges, without protrusions or recesses.

As regards the fastening device (20), in particular for closing the craniotomy hole (21), its use is disclosed in EP-B1-2988687.

At the end of the positioning of the fastening devices (10, 20), the part of the tensioner (12) which projects from the plate (14) after the tightening is removed with the use of a specific cutting device.

Figure 3, in the enlargement of section A-A, shows one of the elastic arms (11), with related small transverse arms (13), which is inserted into the epidural space (X) between the lower surface of the cranial vault (17) and the dura mater (18). As shown in the drawing, the small transverse arms (13) are shaped as a tapered slider, suitable to enable the wedging of the small transverse arms (13) and their following sliding in the epidural space (X) below the dura mater (19), generating only a minimum disconnection of the dura mater (19), and thereby reducing to a minimum the danger of bleeding.

To obtain this result, a particular shape of the tiny elastic arms (11) and of the small transverse arms (13) has been developed. The small transverse arms (13) have a contact surface with the dura mater (19) with a substantially oval shape, and have rounded ends with a geometry designed to make them slide when inserting them in the epidural space (X). The global size of the lower base, composed of tiny elastic arms (11) and small transverse arms (13), ranges from 18.5 x 12.5 mm to 13.5 x 12 mm (preferable value), to reduce the dural disconnection and consequently the risk of epidural hemorraghes.

The tiny elastic arms (11) have an elliptical section with their major axis (w) substantially equap to the width of the craniotomy crack (22) and their minor axis (t) substantially equal to the thickness of the small transverse arms (13).

With such solution, a safe tightening is allowed for the inventive device (10), with really minimum bone thickness sizes, on the order of 0.5 mm.

The small transverse arms (13) have a first and a second bending radius (r₁) and (r₂). The first radius (r₁) is very small, since its purpose is enabling the wedging of the small transverse arms (13) below the dura mater (19). The second radius (r₂) is rather wider, since its purpose is enabling the sliding of the small transverse arms (13) below the dura mater (19).

## Claims

1. Fastening device (10) for fixing a craniotomy operculum to a cranial vault (17), designed to be placed in a craniotomy crack (22), comprising:
- at least one supporting tensioner (12);
- first closing means (11, 13) operatively connected to the supporting tensioner (12);
- second closing means (14) designed to be fastened to the supporting tensioner (12) for completing its closure;
- at least one handle (15) removably connected to the supporting tensioner (12) and designed to handle the device (10) to take it from its rest position to its operating fastening position, wherein the first closing means (11, 13) are connected to a distal end of the supporting tensioner (12) and the second closing means (14) are designed to be fastened to the supporting tensioner (12) in a position near the distal end of the supporting tensioner (12), so that, when the device (10) is in its operating fastening position, the first closing means (11, 13) are placed against the internal distal cranial surface interna and the second closing means (14) are placed against the external cranial surface, the first closing means (11, 13) comprising at least two elongated elastic arms (11) operatively connected to the lower end of the tensioner (12), each of the elongated elastic arms (11) ending in two small elongated arms (13) transversally placed with respect to the elongated elastic arms (11), wherein the two small transverse arms (13) are shaped as a tapered slider, suitable to enable a wedging of the two small transverse arms (13) and their following sliding in the epidural space (X) above the dura mater (19) and the cranial vault, generating only a minimum disconnection of the dura mater (19), reducing to a minimum the danger of bleeding, the small transverse arms (13) having a contact surface with the dura mater (19) with a substantially oval shape, and having rounded ends with a geometry designed to make them slide when inserting them into the epidural space (X), **characterised in that** the elongated elastic arms (11) have an elliptical section.

2. Fastening device (10) according to claim 1, **characterized in that** the global size of the lower base, composed of tiny elastic arms (11) and small transverse arms (13), ranges from 18.5 x 12.5 mm to 13.5 x 12 mm.

3. Fastening device (10) according to claim 1, **characterized in that** the elliptical section of the elongated elastic arms has a major axis whose length is equal to the width (w) of the craniotomy crack (22).

4. Fastening device (10) according to claim 1, **characterized in that** the elliptical section of the elongated elastic arms has a minor axis whose length is equal to the thickness (t) of the small transverse arms (13).

5. Fastening device (10) according to claim 1, **characterized in that** the small transverse arms (13) have a first bending radius (r₁) and a second bending radius (r₂), the first bending radius (r₁) being much shorter than the second bending radius (r₂), the first bending radius (r₁) having the purpose of enabling the wedging of the small transverse arms (13) below the dura mater (19), the second bending radius (r₂) having the purpose of enabling the sliding of the small transverse arms (13) below the dura mater (19).

## Patentansprüche

1. Fixierungsvorrichtung (10) zur Fixierung eines Kraniotomieoperculums an einem Schädelgewölbe (17), das zur Platzierung in einem Kraniotomieschlitz (22) vorgesehen ist, umfassend:
- mindestens einen Stütztensor (12);
- erstes Schließmittel (11, 13), das betriebsmäßig mit dem Stütztensor (12) verbunden ist;
- zweites Verschlussmittel (14), das zur Befestigung am Stütztensor (12) ausgelegt ist, um dessen Verschluss zu vervollständigen;
- mindestens einen Griff (15), der entfernbar mit dem Stütztensor (12) verbunden ist und dazu ausgelegt ist, die Vorrichtung (10) zu manipulieren, um sie aus ihrer Ruheposition in ihre operative Befestigungsposition zu bringen, in der das erste Schließmittel (11, 13) mit einem distalen Ende des Stütztensors (12) verbunden sind und das zweite Verschlussmittel (14) so ausgelegt ist, dass es an dem Stütztensor (12) an einer Position nahe dem distalen Ende des Stütztensors angebracht ist. Stütze (12), so dass, wenn sich die Vorrichtung (10) in ihrer operativen Fixierungsposition befindet, das erste Schließmittel (11, 13) gegen die innere distale Schädelfläche und das zweite Schließmittel (14) gegen die äußere Schädelfläche gelegt werden; das erste Schließmittel (11, 13) umfasst mindestens zwei längliche elastische Arme (11), die betriebsmäßig mit dem unteren Ende des Tensors (12) verbunden sind, wobei jeder der länglichen elastischen Arme (11) in zwei kleinen endet die länglichen Arme (13) sind quer zu den länglichen elastischen Armen (11) positioniert, bei denen die beiden kleinen Querarme (13) eine sich verjüngende Schuhform aufweisen, die geeignet ist, das Verkeilen der beiden kleinen Querarme (13) und zu begünstigen das anschließende Gleiten in den Epiduralraum (X) über die Dura Mater (19) und das Schädelgewölbe, was nur eine minimale Ablösung der Dura Mater (19) bewirkt und das Blutungsrisiko der kleinen Querarme (13) minimiert eine Kontaktfläche mit der Dura mater (19) von im wesentlichen ovaler Form und mit abgerundeten Enden mit einer Geometrie, die sie in die Einfügung in den Epiduralraum (X) gleiten soll;
**dadurch gekennzeichnet, dass** die länglichen elastischen Arme (11) einen elliptischen Querschnitt haben.

2. Befestigungsvorrichtung (10) nach Anspruch 1, **dadurch gekennzeichnet, dass** die Gesamtabmessung der unteren Basis, bestehend aus elastischen Armen (11) und kleinen Querarmen (13), von 18,5 x 12,5 mm bis 13,5 x 12 mm variiert.

3. Befestigungsvorrichtung (10) nach Anspruch 1, **dadurch gekennzeichnet, dass** der elliptische Abschnitt der länglichen elastischen Arme eine Hauptlängenachse aufweist, die der Breite (w) des Kraniotomieschlitzes (22) entspricht.

4. Befestigungsvorrichtung (10) nach Anspruch 1, **dadurch gekennzeichnet, dass** der elliptische Abschnitt der länglichen elastischen Arme eine kleine Längsachse aufweist, die der Dicke (t) der kleinen Querarme (13) entspricht.

5. Befestigungsvorrichtung (10) nach Anspruch 1, **dadurch gekennzeichnet, dass** die kleinen Querarme (13) einen ersten Krümmungsradius (r1) und einen zweiten Krümmungsradius (r2) aufweisen, wobei der erste Krümmungsradius (r1) ist viel kleiner als der zweite Krümmungsradius (r2), wobei der erste Krümmungsradius (r1) den Zweck hat, das Verkeilen der kleinen Querarme (13) unter der Dura Mater (19) zu begünstigen, der zweite Krümmungsradius (r2)) mit dem Ziel, das Gleiten der kleinen Querarme (13) unter die Dura mater (19) zu begünstigen.

## Revendications

1. Dispositif de fixation (10) pour la fixation d'un opercule de craniotomie à une voûte crânienne (17), conçu pour être placé dans une fente de craniotomie (22), comprenant:
- au moins un tenseur de support (12);
- des premiers moyens de fermeture (11, 13) reliés fonctionnellement au support tenseur (12);
- des seconds moyens de fermeture (14) destinés à être fixés sur le support tenseur (12) pour achever sa fermeture;
- au moins une poignée (15) reliée de manière amovible au support tenseur (12) et destinée à manipuler le dispositif (10) pour l'amener de sa position de repos à sa position de fixation opérationnelle, dans laquelle les premiers moyens de fermeture (11, 13) sont reliés à une extrémité distale du tenseur de support (12) et les seconds moyens de fermeture (14) sont conçus pour être fixés au tenseur de support (12) à une position proche de l'extrémité distale du tenseur de support. support (12) , de sorte que, lorsque le dispositif (10) est dans sa position de fixation opérationnelle, les premiers moyens de fermeture (11, 13) sont placés contre la surface crânienne distale interne et les seconds moyens de fermeture (14) sont placés contre la surface crânienne externe, les premiers moyens de fermeture (11, 13) comprenant au moins deux bras élastiques allongés (11) reliés fonctionnellement à l'extrémité inférieure du tenseur (12), chacun des bras élastiques allongés (11) se terminant par deux petits les bras allongés (13) positionnés transversalement par rapport aux bras élastiques allongés (11), dans lesquels les deux petits bras transversaux (13) ont une forme de chaussure effilée, apte à favoriser le coincement des deux petits bras transversaux (13) et leur glissement ultérieur dans l'espace péridural (X) au-dessus de la dure-mère (19) et de la voûte crânienne, ne provoquant qu'un décollement minimal de la dure-mère (19), minimisant le risque de saignement, les petits bras transversaux (13) ayant une surface de contact avec la dure-mère (19) de forme sensiblement ovale, et ayant des extrémités arrondies avec une géométrie conçue pour les faire glisser dans l'insertion dans l'espace péridural (X),
**caractérisé en ce que** les bras élastiques allongés (11) ont une section elliptique.

2. Dispositif de fixation (10) selon la revendication 1, **caractérisé en ce que** la dimension hors tout de la base inférieure, constituée de bras élastiques (11) et de petits bras transversaux (13), varie de 18,5 x 12,5 mm à 13,5 x 12 mm.

3. Dispositif de fixation (10) selon la revendication 1, **caractérisé en ce que** la section elliptique des bras élastiques allongés a un grand axe de longueur égale à la largeur (w) de la fente de craniotomie (22).

4. Dispositif de fixation (10) selon la revendication 1, **caractérisé en ce que** la section elliptique des bras élastiques allongés a un petit axe de longueur égale à l'épaisseur (t) des petits bras transversaux (13).

5. Dispositif de fixation (10) selon la revendication 1, **caractérisé en ce que** les petits bras transversaux (13) ont un premier rayon de courbure (r1) et un deuxième rayon de courbure (r2), le premier rayon de courbure (r1) étant beaucoup plus petit que le deuxième rayon de courbure (r2), le premier rayon de courbure (r1) ayant pour but de favoriser le calage des petits bras transversaux (13) sous la dure-mère (19), le deuxième rayon de courbure (r2) ayant pour but de favoriser le glissement des petits bras transversaux (13) sous la dure-mère (19).
